# EUROPEAN PATENT APPLICATION

(11) **EP 4 759 346 A1**
(43) Date of publication of application: **17.06.2026**
(21) Application number: 24307073.7
(22) Date of filing: 11.12.2024
(51) Int. Cl.: A61M 39/10, A61M 39/18

(54) **GENDERLESS ASEPTIC CONNECTING DEVICE FOR FLUID CONNECTION WITH CONTROLLED MEMBRANE PULL AND CONNECTION ARRANGEMENT USING A PAIR OF CONNECTING DEVICES**

(71) Applicant: Sartorius Stedim FMT, 13400 Aubagne (FR)
(72) Inventor: DELASALLE, Brice, 13600 LA CIOTAT (FR); BRAUN, Michael, 37127 DRANSFELD (DE); BLAKE, Florian, 13600 LA CIOTAT (FR)
(74) Representative: Plasseraud IP

(57) **Abstract**

The connector (1) has a tubular body (2) with a flange (3), forming a central opening between a female member and a male member having a retaining tab (4r). A membrane (M) removably attached to the connector covers this face to seal the opening and a genderless connecting device is obtained, allowing mating engagement of the connector (1) with an identical complementary connector. An interface in the flange allows a slider coupled to the membrane to be retained. The slider (10) cooperates with a slide along a sliding direction perpendicular to the body axis (A2), the slide belonging to the flange (3) and providing a guiding length greater than a diameter of a seal surrounding the opening. This prevents any slider deviation before a membrane closing portion (CM) is displaced away from the opening (O), when performing a sliding disengagement of the slider and pulling the membrane using the slider.

## Description

### FIELD OF THE INVENTION

The instant disclosure generally relates to fluid management systems in the field of biopharmaceutical applications, including locking components for efficiency of fluid circulation, and more particularly to a fluid handling coupling or connector that can allow a quick connection, while being genderless, and to an aseptic coupling arrangement using a pair of such connectors.

### TECHNICAL BACKGROUND

In the pharmaceutical, chemical field, that uses tubing and assembling of components in the processing and transport for fluids (such as biopharmaceutical fluids), there is often a need for aseptic connection, at least for the first connection. Hoses carrying biopharmaceutical fluid may be commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field.

Aseptic coupling devices can be used to connect two or more sterilized pathways. For example, aseptic connectors can be used to couple a fluid pathway from a first piece of processing equipment or container to a fluid pathway from a second piece of processing equipment or container to establish a sterile pathway for fluid transfer therebetween.

Generally, aseptic connectors require a dry connection obtained using clamping devices placed upstream of the aseptic connectors so that the aseptic connectors are kept free of fluid while the connection between the aseptic coupling devices is made. After establishing the sterile connection between the connectors, the clamping devices are removed to allow fluid to flow through the aseptic connectors.

On aseptic connectors, asepticity is often obtained by means of a flexible membrane. Aseptic membranes are usually each welded to a corresponding front face of the connector and can be pulled tangentially to the mating face when connectors are paired, after a suitable coupling operation. The membrane can be provided with a fold or folding line, at the opposite from the pulling side, such folding line being typically rectilinear and defining a junction between:
- a sealing/closing portion adhering with an annular surface provided at the front of the connector, so that the connector opening is hermetically closed in aseptic manner by the closing portion,
- and an overlapping membrane portion, overlapping the closing portion and extended by a pulling part provided at the pulling side.

Current connectors may be of complex conception, requiring assembling steps or handling operations before practically obtaining the fluid connection. Some connection systems require an additional interface forming a female interface for the two devices forming a male connector. Other connections systems imply a combination of an axial coupling and rotation of sleeve or collar to lock the connection.

In biotechnology and bioprocessing field, vessels have new construction adapted for single use applications. There is a need for minimizing complexity and amount of plastic material of aseptic connectors, while ensuring sterility and robustness of the connections, typically using shieldable flanged connecting devices that have each a removable membrane (to shield the proximal/front opening of the connector).
Indeed, to prevent the content within tubes from escaping while connecting two or more tubes, sterile genderless interface connectors can be useful to connect the tubes at the point of connection. A genderless interface connector includes a membrane at the interface inside the connector and a tab outside the connector that is attached to the membrane. The membrane prevents fluid connection between the connected tubes at the interface. After the genderless interface is securely engaged with the tubes on each side, the tab can be pulled away from the connector to remove the membrane from the interface so that the tubes are connected. In this way, sterility is maintained in the tubes and the connection does not contaminate the environment.

Documents US 2003/0030272 and EP3488895 disclose a disposable aseptic connection system that is a disposable connection. One of the main risks of the operation is to damage and tear the membrane which could breach the asepticity. Practically, pulling the tab by hand has ergonomic implications because of the force and impact stress induced throughout the process. Depending on the gesture, the membrane may be partially removed or torn, causing damage to the sterile condition. Furthermore, if significant force is applied to pull the tab, this can lead to dislocation of the genderless interface connector, further compromising the sterility of the environment. Besides, this kind of connector has latching means provided with flexible clips, which are quite fragile parts.

More generally, there is still room for improvement regarding robust constructions of fluid connectors, in a way optimizing the way of operating with such fluid connectors, in aseptic conditions and/or in user-friendly conditions.

### OBJECTS AND SUMMARY

For improving situation, embodiments of the invention provide a connecting device for fluid connection, having a connector (possibly genderless) and a membrane, the connecting device comprising:
- a tubular body extending around a longitudinal axis of the connector and delimiting an interior space (for allowing fluid passage), the tubular body having an opening at a front of the tubular body where the interior space axially opens;
- a flange formed peripherally on the tubular body around the interior space, the flange comprising a female connecting member and/or a male connecting member (protruding axially from a flange front face to provide a first insert having a fastening region);
- an aseptic attachment structure including the flange front face and the membrane that is removably attached to the connector at the flange front face to seal the opening;
- an annular seal element providing an annular contact surface at the flange front face, radially interposed between the opening (typically forming a central opening at the front of the connector) on the one hand, and the female connecting member and/or a male connecting member (or any similar interlocking part arranged on the flange) on the other hand; and
- a slider mounted on the flange to be movable relative to the flange, along a direction transverse to the longitudinal axis;
   wherein the membrane is secured to an external section of the slider and comprises a closing portion axially covering and sealing the opening;
   wherein the slider is in a predefined engaged position relative to the flange and comprises:
      - the external section that extends outside the flange in the predefined engaged position and preferably laterally covers the flange (in this engaged position); and
      - one or two fingers, each rigid and protruding inwardly from the external section, parallel to the closing portion and perpendicular to the longitudinal axis, allowing the slider to be retained, in said predefined engaged position, in a slide that allows sliding engagement and sliding disengagement of said one or two fingers along a sliding direction perpendicular to the longitudinal axis, the slide being provided as an integral part of the flange,
   and wherein a guiding length of the slide, which may be superior or equal to an internal or external diameter of the annular seal element (or possibly superior to the opening diameter), is sufficient to prevent any rotation of the slider relative to the flange and any slider deviation at least before the closing portion is away from the opening, when performing the sliding disengagement and pulling the membrane by a pulling action using the slider.

Advantageously, an elongated slide is thus formed directly by the flange, which facilitates disconnection of the membrane in a proper way: the membrane pulling can be systematically performed in the optimal direction in a reproductible way, regardless of the specific operator performing the membrane removal. Since the slider is initially retained and cannot quickly deviate relative to the flange, risk of not pulling well the membrane is efficiently prevented. The finger can be longer than the external diameter of an annular seal element housed by the flange and surrounding the opening. An inserted section of the finger (inserted in flange elongated cavity or similar hollow part of the slide) can be longer than such external diameter of the annular seal element.

Typically, the slide is provided with hollow guiding parts of constant section (keeping the same guiding profile) and/or that are not tapering, typically using two opposite parallel surfaces to guide at least one finger of the slider. More generally, the slide and the one or two fingers are of complementary shapes, suitable to have the guiding parts of the slide preventing any deviation. The slide can define a constant spacing at a guiding part and/or a constant spacing between two spaced guiding parts. Preferably, the slider finger received in a guiding part is typically constructed with two opposite side parts that extend parallel, with this finger fitted to a particular guiding part of the slide. When having two fingers and two spaced guiding parts, the sections of the two fingers of the slider can preferably be different to prevent any wrong insertion, with each access for the associated guiding parts also different in cross section.
When having two fingers, a good stabilizing effect is obtained. The fluid connection, obtained in a connection assembly involving two identical connectors (genderless) or two mating connectors, the pulling can be performed along a constant direction to simultaneously remove the two membranes (placed adjacent due to a mechanical coupling involving the male and female connecting members). Since finger(s) form anti-rotation means that are distributed in the two connecting devices, an inadequate pulling of the closing portion/member is prevented. The membrane is partly covering the front face and can be radially extended by the external section of the slider. In a suitable manner, the (aseptic) connecting device as above recited is a first device with the external section of the slider being a first gripper half or part configured to be attached to a second gripper half or part of a second (aseptic) connecting device, identical typically, such that the membrane of the first device and a membrane of the second device can be removed, simultaneously.

The membrane can have a distal margin portion, provided at the opposite from the external section of the slider and adjacent to the membrane closing portion covering the opening, with limited radial or transversal size, typically inferior to half diameter or to diameter of the opening (a radial size less than 50 or 60% of the opening diameter can be preferred and/or a radial size less than 10 or 12 mm for the distal margin portion, the opening diameter possibly being less than 19 or 21 mm). The distal margin portion extends along the pulling direction (corresponding to the sliding direction) between the closing portion and a membrane end edge that can optionally extend perpendicular to the longitudinal axis.
The guiding length of the slide, superior to the opening diameter, can exceed the opening diameter with an excess in length superior to the radial size of the distal margin portion.
A proximal portion of the membrane (at the opposite from the distal portion) may be provided to form a short intermediate part between the closing portion and the rigid external section of the slider. A developed length of the intermediate part can be shorter (possibly twice shorter) than the external diameter of the seal/seal element and/or not exceeding 1,5 cm.

The membrane can have a pulling portion, formed as an extension of the closing portion or provided as an extension of an overlapping portion that overlaps the closing portion. The pulling portion may be in direct contact with a gripper or similar part formed by the external section of the slider. The pulling portion can be covered and/or stiffened by the external section so that the pulling portion cannot be folded or cause any membrane deviation during the pulling operation, at least as long as the closing portion remains attached to the flange to seal the opening (the membrane attachment at the flange front portion being possibly performed after the slider is anchored to the flange with finger(s) properly inserted).
In the aseptic attachment structure (with the connector front face covered by the membrane that is removably attached to the connector front face), the membrane is peelable and removably attached (directly) to the flange front face to seal the opening, typically with the membrane overlapping the seal element and adhering only with a flange front surface.

The slide can include a housing, or a pair of separate housings provided inside the flange. The connecting device is genderless, due to the structure at the front of the flange and with the external section that can be a radial part, preferably without protruding beyond the flange front face when two connecting devices are coupled to reach a pre-coupled state ready for membrane removal. Besides, the external section of the slider can have a width equal or superior to a width or maximum width of the membrane and the slider may be provided without any piece rotating around the longitudinal axis, typically with no rotation gesture required once the connecting device is facing a compatible connecting device.
The external section is provided with a slider extension that forms a gripper (gripper shaped extension), preferably having a fastening face to be a gripper half adapted to be combined with an identical or complementary slider extension provided in a complementary connecting device.

In some embodiments of the connecting device, the slider and the slide have interlocking means that allow locking the retained position (fully inserted position) of the slider. Additionally, or alternatively, the following dispositions can be adopted:
- the slide comprises two guiding parts, one of which being a cavity extending, inside the flange.
- one of the two guiding parts is a cavity inside the flange, which extends from a mouth opening at a first side of the flange, allowing insertion of an elongated finger that forms all or part of the one or two fingers, to a cavity end at a second side of the flange.
- the cavity end extends at a second side of the flange, which is provided at the opposite from the first side, with the elongated finger being longer than the diameter of the opening and/or having a finger inserted section (inserted in the cavity) longer than an external diameter of an annular seal element provided around/surrounding the opening, to have a finger end extending in the cavity end.
- the one or two fingers comprise an elongated finger configured to be linearly moved in the slide.
- the elongated finger is preferably movable between a front wall and a rear wall of the flange.
- the elongated finger is preferably movable between two parallel guiding parts or surfaces of the slide.
- the two parallel guiding parts or surfaces can be included in front and rear walls of the flange or they can be provided perpendicular or transverse to the front and rear walls of the flange.
- the elongated finger is linearly guided by the two parallel guiding parts and flange walls (rear and front walls) that prevent any transverse deviation with the sliding direction maintained by the guiding parts or surfaces (the elongated finger is thus prevented to rotate during the sliding disengagement).
- the elongated finger is guided by at least one amongst a flange rear wall and a flange front wall that are parallel walls between which the two parallel guiding parts extend to prevent any rotation and deviation of the elongated finger during the sliding disengagement.
- a sliding finger (possibly the elongated finger as above mentioned) of the slider can optionally include a hollow part or a groove, delimited/arranged between two elongated sides of the elongated finger.

The flange may be provided with one or more of the following features:
- the flange has four sides of a rectangular side wall.
- a cavity, preferably a peripheral cavity delimited/closed by a given side of the external side wall, is provided in the flange.
- the cavity extends from a mouth opening at a first side of the flange to a cavity end that extends at a second side of the flange.
- the interior space is interposed between the first side and the second side of the flange.
- the elongated finger is received inside the flange in the cavity.
- the cavity has a cross-section sized and shaped for linearly guiding the elongated finger, the cavity being delimited by a rail, preferably a rail having U-shaped profile.
- the cavity communicates (typically at a side of that is not delimited by the rail/any of the three guiding walls of the rail) with a hollow portion of the flange that is in axial alignment with an axial access provided for a female connecting member of the flange.
- the flange is provided with an inner shoulder or abutment surface that is facing axially away from the external section of the slider.
- the flange may have two opposite outer sides that are parallel sides, possibly extending substantially rectilinear.

The slider may be a single piece or any suitable assembly, movable parallel to the two opposite outer sides, transversely and perpendicular to the membrane sealing the opening.

In some options, the slider has latching means formed on or that co-act with the one or two fingers lock and retain the slider in the predefined engaged position. Preferably, the latching means can engage in the hollow portion of the flange that is (possibly) provided adjacent to the guiding part where a finger is inserted with a guiding effect.
The guiding effect is completed by a retaining effect, for instance using the inner shoulder or abutment surface or any suitable detent member. An abutment contact is obtained between the latching means that are inserted in the flange when inserting the finger(s) in the slide.

The slider is configured to be rigid in the external section, at least in a portion of the external section carrying the one or two fingers. In preferred embodiments, the slider has a rigid structure and/or is a single piece of plastic material. The slider can be provided with the following features:
- the slider is molded from a plastic material, preferably a thermoplastic material.
- the slider comprises a contacting wall having a given height, possibly at least equal to a thickness of the flange, the contacting wall being in contact with the external side wall of the flange.
- the external section of the slide has thickness (measured parallel to the longitudinal axis) that is lower than height of the contacting wall.
- the slider is provided with a gripper or gripping portion, at a free end of the external section, with a local increase or progressive increase in thickness with increased distance from the flange.
- two fingers are provided with one finger wider than the other (width size being measured along a transverse direction perpendicular to the longitudinal axis), possibly with the wider finger being also comparatively longer

In the slider, the latching means can be deformable or displaceable, preferably with displacement along a direction perpendicular or distinct from the sliding direction of the slider, under a pushing action to unlock the slider (the pushing action being thus typically exerted along a pushing direction transverse to the sliding direction).
The latching means may be arranged, as considered along the pulling direction, closer/proximal to the external section and distal/away from a free end of the elongated finger. A latching part shorter than the elongated finger can be provided, so that flexing of the latching part can start after a preliminary guiding effect, possibly already a linear guiding, is obtained by a partial insertion of the elongated finger in the slide.

According to embodiments the slider can have at least one of the following features:
- the external section is wider than a complementary insertable section/part of the slider.
- the at least one sliding finger comprises two elongated fingers that extend parallel, the interior space extending between the two elongated fingers.
- the two elongated fingers are configured to be linearly moved in the slide.
- the slider has two fingers that form part of a U-shaped stabilizing part of the slider.
- the two fingers (spaced by an interspace where the longitudinal axis extends) cooperate with two rails, the slide including the two rails that are preferably formed by a single piece (hollow piece that may constitute the flange).
- the stabilizing part comprises the slider guiding means, which include the two fingers (parallel fingers), and a contacting wall of the external section, which extends transverse to a direction of the two fingers.
- the stabilizing part can include a latching means/part to lock the predefined engaged position.

In some options, a latching means can be provided adjacent to or separate from the one or more fingers of the slider. The retaining of the slider can be performed by co-action of the latching means/part and the latching means.

In the connecting device, the seal element (possibly of an elastomer material) is providing an annular contact surface at the flange front face, this surface being typically covered by an annular margin of the closing portion of the membrane. The seal element can be interposed radially between:
- the opening on the one hand,
- and an axial access of the female connecting member and the male connecting member on the other hand.

The female connecting member may be provided with a locking region and an axial access, the axial access preferably opening at the front of the tubular body.

A virtual plane passing through the slide, which is a plane perpendicular to the longitudinal axis, can intersect the seal element, while being axially offset relative to the front face of the flange. Optionally, each guiding part of the slide, configured to receive a corresponding finger of the slider, can have a rail with a U-profile or with a rectangular section.

In some options, the connector is genderless and thus can be in a pre-coupled configuration with a complementary identical connector, with the membrane sandwiched between the flange front faces of the two connectors.
Typically, the female connecting member is provided with a locking region and an axial access, the axial access preferably opening at the flange front face,
and wherein the slide length is greater than a length of the male connecting member as measured from the flange front face.

In options, the flange can have the female connecting member provided with one or more of the following features:
- a retaining surface provided inside the flange and an axial access for an insert of the mating male connecting member, so that the retaining surface can be engaged by a radial protrusion of this insert, in a connection configuration.
- the axial access allows insertion of a second insert, of same structure as the first insert and thus also having a fastening region, the second insert being part of an identical complementary connector genderless for the fluid connection and being sized and shaped for engagement of the retaining surface with the fastening region of the second insert, in a connection configuration.

According to some options, the slider includes a resilient member having a latching part configured to latchingly engage an abutment surface of the flange (distinct from the retaining surface), which is a surface preferably provided within an inner cavity of the slide, to prevent the sliding disengagement.
In the connection configuration with the second insert engaged, the latching part is pushed toward a disengaged insertion position allowing the sliding disengagement (the disengaged insertion position being a position with the latching par offset relative to the abutment surface). The pushing may be an axial pushing and/or a radial pushing. This pushing action is allowed due to flexibility of the latching part (possibly with a flexibility of the finger as a whole when the latching part is carried by the finger)) and presence of an empty space adjacent to the finger, where the latching part can be shifted/displaced.
Typically, in a connection assembly having two connecting devices mutually coupled, each slide allows a snap fit tab or similar latching part to be engaged/retained, for instance along a guide rail/guiding part of the slide. The latching part interferes with the engagement (insertion) of the insert in the mechanical interlocking of the connecting devices, so that it is pushed, for instance just before finalizing the interlocking with wo inserts properly engaged on corresponding retaining surfaces.

As soon as the connecting devices are matingly engaged, each slider mounted on a flange becomes ready to be disengaged, due to the release obtained for the latching part. More generally, it is understood that each slide provides a locking region that can be unlocked by use of the other connecting device (typically by a male member thereof that protrudes axially outward with respect to a flange front face).

According to embodiments of the connecting device, the following dispositions or options can be adopted:
- the external section has covering/contacting wall and a handle plate part extending radially outward, from the covering/contacting wall that is in contact with the external side wall of the flange.
- the handle plate part is a half of a graspable portion and is provided with a protruding rim protruding axially from the handle plate part, away from a junction plane.
- the external section has an inner face that extends between the two parallel fingers of the slider the inner face being in contact with the external side wall.
- the inner face is bulged inwardly to form a bulged part.
- the inner face laterally covers the flange in the predefined engaged position/
- the inner face is in contact with the external side wall at a seating surface that comprises a recess, while at least one contact area is obtained between the external section and the seating surface.
- the external section of the slider has the bulged part filling or being engaged in the recess, whereby the external section has a surface, facing opposite from the closing portion membrane, which is arranged centrally in the external section and inwardly (radially) delimited by a protruding wall performing the contact area.
- the surface arranged centrally provides a finger contact area adjacent to a bottom region of the recess.
- the finger contact area allows a finger to be placed close to the flange, and possibly offset radially (beyond the front face of the flange) to reach a position close to the junction plane of the two membranes, when two connecting devices are coupled (in an assembly providing the fluid connection).

In some options, the elongated finger is connected to the external section at a junction that is:
- at a distance from the bulged part; and
- more distant from the opening than the protrusion part.

The connector of the connecting device, preferably provided with a genderless construction with a male connecting member and a female connecting member around the opening in the flange, can have:
- the retaining surface configured to axially retain the second insert at the fastening region thereof in a locking configuration of the fluid connection, the second insert being formed in the complementary connector that is suitable to be coupled to said connector for enabling the fluid connection.

The locking configuration is typically obtained when the second insert passing through the axial access is inserted in the female connecting member to have a second insert end extending axially beyond the retaining surface. In such locking configuration (with pre-coupled state obtained), the female connecting member can advantageously delimits an insertion channel between:
- an external wall side part of the flange that includes the retaining surface,
- and a guiding part of the slide where a finger of the slider extends, possibly extending as far as or beyond a membrane edge that is at the opposite from the external section. Alternatively, the insertion channel is delimited between:
- a groove or region housing the sela element,
- the external wall side part of the flange that includes the retaining surface (with the guiding part of the slide, where a finger of the slider extends, possibly being offset radially outward relative to the insertion channel (the finger may extend as far as or beyond a distal membrane edge).

The latching part is provided with a pushable surface located inside or adjacent to an insertion channel delimited by the female connecting member, whereby the disengaged insertion position of the latching part is obtained upon pushing the pushable surface by the second insert to have the locked configuration of the fluid connection.
Optionally, the male connecting member, inserted through the axial access, forms the only part protruding beyond the flange front face. Alternatively, the male connecting member forms a first protruding part of the connector protruding axially from the flange front face, while a second protruding part of the connector, protruding axially beyond the flange front face, possibly playing the role of an unlocker configured to unlatch a retaining end or similar part of the slider, that engages with an abutment surface or rim provided in the slide.

The slider is configured to be transversely moved, along a first direction perpendicular to the longitudinal axis with provision the second insert has been inserted via the axial access. Preferably, the slider is snap fit, possibly in an inner region of the flange, which is a region in alignment with the axial access along a direction parallel to the longitudinal direction. The slider may have a finger lug to allow the snap fit (with the finger lug reaching the inner region aligned with the axial access).
A clipping of the slider can be obtained due to the elastic return force of the latching part that can be separated from an adjacent finger by a slot. A shorter size of the latching part can be preferred, so that deformation of the latching can start only after the slider is guided without any deviation possibility when complementing/finishing the sliding engagement.

According to a particular option, the connector is a first aseptic coupling device for coupling to a second aseptic coupling device consisting in the identical complementary connector and an associated membrane, the membrane of the first aseptic coupling device being a first peel off membrane removably coupled to and at least partially covering the flange front face and the seal element. The flange of the connector, preferably formed in one piece with the tubular body, has an external side wall that can have a perimeter with four sides that delimit a square or a rectangle. The slider can be partly housed in the housing or recess. The axial access may be adjacent to a guiding channel of the slide.

Typically, the closing portion has a given attachment extension along the sliding direction, which may be optionally inferior to maximum size of the flange along the pulling direction. The flange of the connector has along the sliding direction an external size, delimited between two opposite parallel surfaces of the external side wall, superior to the given attachment extension. Advantageously, the guiding length of the slide is superior or equal to the given attachment extension (which may be a width or characteristic size of a rectangular attachment surface where the closing portion adheres with the flange front surface (around the opening).
A guiding length superior or equal to 2.5 or 3.5 cm can be preferred (superior to 1 inch, i.e. 2.54 cm for instance). The attachment surface may be inferior or equal to 10 or 12 cm². With or without such surface area for the attachment surface for the closing portion, the guiding length can be superior or equal to 2.5 cm, for instance comprised between 3 and 7 or 8 cm.

In some embodiments, the external section laterally covers the flange, being for instance configured to rest against a seating surface provided at an external side wall of the flange. Optionally, the seating surface comprises a concave surface facing radially outward and delimited between two guiding end walls that are perpendicular to the flange front face. One of these guiding end walls may guide a first finger of the slider and the other is guiding a second finger of the slider. These two fingers are thus distributed on either side of the slider and a central bulged part can be provided to engage the concave surface.

The slide can be formed rearwardly, with respect to the membrane or the flange front face, while the fastening region of the first insert can typically extend, axially, at a given distance from the flange front face. This given distance (axial distance) can be more than axial distance, in the female connecting member, between the front face and the retaining surface.

When the connecting device is genderless, operations are user-friendly and the connecting device can satisfy all the needs of a modular aseptic connection and possibly disconnection, with a peel-off membrane, an axial mating and sealing direction and using a clipping or similar latching that can be realized in an inner locking region, thus preventing any fragile parts to be broken.

The flange can form a slide for the slider.

In some options, embodiments of the connecting device provide one or more of the following features:
- the first insert comprises an inserting tab that is shifted/offset on a first side relative to the longitudinal axis.
- a clipping is obtained when engaging the second insert with the retaining surface, while a clipping may also be obtained, simultaneously, when engaging the first insert with the retaining surface of the connector of the other connecting device.
- due to the slight excess of the given distance (axial distance) as compared to the positioning of the retaining surface, a gap exists in coupled state between the two frontal (also called proximal) front faces of the flanges, so that the membranes can be extracted through this gap, using simultaneously two pull portions of these membranes distributed in the two identical connecting devices.
- each membrane is thinner than a half size of the gap and/or can be pulled and slide within this gap, starting from a pre-coupled state with the two identical connecting devices coupled before any removal of membrane.
- the flange front face is planar/substantially planar.
- the male connecting member forms the only part of the connector protruding axially from the flange front face.
- the insert (first insert) may be a single protruding member of the connector, which protrudes from the front face with a protruding extension greater than a depth (or longitudinal extension) of the axial access provided by the female connecting member.
- a complementary of shape/circumference may be used for having the second insert filling the axial access.
- the insert may be part of a group of parallel inserts that either engage into a single opening or slot forming the axial access, or engage in different openings available on the flange (each of the different openings at the flange front face).
- the membrane may have no part overlapping the external side wall and is suitable to be pulled radially along a pulling direction.

Possibly, the seals may of simple shape, for instance without requirement for any transverse portion protruding radially outward and/or externally covering the flange front face. Each of the seals may have an expanded state, to have as seal length that is superior to a depth of a front groove housing the corresponding seal, whereby the seals can be mutually in axial contact outside the grooves, within the gap. No rotation of the seals is required, once the first and second inserts are facing the corresponding axial accesses of the female connecting members.

In some options, a secondary slider may be provided to form for locking the coupling of the two connectors. The secondary slider may include a piece distinct from the flange and distinct from the slider can optionally be operable by a manual actuation using an external actuator or push-button, preferably arranged to radially protrude outward relative to the flange. The manual actuation of the secondary slider may cause an interfering portion of this secondary slider to be moved radially to engage and stop an insert tab (of the male connecting member) passing through the axial access of the female connecting member.

In embodiments of the connecting device with a flexible member or tab provided in the first insert, one or more of the following features can be provided:
- the flexible member is forming at least one relief, while the second insert also has at least one relief in a flexible member.
- the flexible member allows a clipping, when engaged with the retaining surface.
- the flexible member has a protrusion extending in the slide in an engaged state (clipped state, for instance) of the corresponding insert.
- the male connecting member is an unlocking member for release of the slider that is no more engaged in the anchoring region of the flange.
- the unclipping action to release the slider (unclipping obtained by a snap fit of the flexible member of the second insert) is allowed just before or as soon as the at least one relief has been engaged with the retaining surface provided in the female connecting member (in the flange).

In each connecting device, the closing portion belonging to the membrane is covering the opening in the pre-coupled state. The pre-coupled state is a state, in which the locking configuration is established for axial retaining of the male connecting members (two inserts distributed in two connectors when having genderless connectors), so that two closing portions are in alignment and/or in an overlapped configuration. In the pre-coupled state (typically after properly inserting the second insert in the female connecting member), the closing portion is adapted to be removed together with the other closing portion, by using synchronizing means that are provided in the two sliders to mutually fasten the pull portions of the membranes or any other portion(s) of the membranes that extend beyond the flanges. The fingers distributed in the two sliders thus can be disengaged simultaneously with exactly the same sliding direction when disengaging a graspable portion in common for the two sliders

Whatever the way the locking configuration is obtained, the slide or a housing provided with the slide may open radially outward, through an external side wall of the flange, to allow positioning and/or controlling the slider. In some embodiments facilitating pushing and pulling of the slider, one or more of the following features are provided:
- the external section has a pullable portion provided outside the flange;
- the slider is configured to be transversely pulled, only along a direction perpendicular to the longitudinal axis once insertion of the second insert via the axial access has been performed,
- before any connection, the slider is clipped inside the flange, without being permanently retained in the housing due to latching means/part carried by the external section or a slider finger.
- a latching part of the slider is a securing clip, configured as a tab of smaller length than the elongated finger, preferably concealed by the external side wall and pushable via the axial access provided at the flange front face.
- the clipping or snapping of the slider is obtained by axially retaining a lug included in the latching part, the lug facing in direction opposite to an insertion direction of the connecting male member that is inserted via the axial access in the female connecting member.

To obtain a connected state with the connector assembled (coupled) with a compatible connector of same structure, with the membranes ready/adapted to be removed to pass from a pre-coupled state to a coupled state, one or more of the following features are provided:
- the connector is a first aseptic coupling device for coupling to a second aseptic coupling device, consisting in the identical complementary connector and an associated membrane.
- the membrane of the first aseptic coupling device is a first peel off membrane removably coupled to and at least partially covering the flange front face and the seal element.
- in pre-coupled state of the first and second aseptic coupling devices, the first membrane has a pull end part secured to the first slider, preferably including a coupling portion with at least one rigid member, couplable with a complementary/second slider to which is secured a pull end part of the membrane of the second aseptic coupling device, which is a second peel off membrane.
- the slider may include at least one snap or axial pin configured to be coupled to a slider of the other aseptic coupling device.

According to another aspect, embodiments provide an aseptic coupling arrangement, comprising a first coupling device and a second coupling device, which are each provided with the connecting device according to any one of the preceding claims, the axial access of the first coupling device allowing insertion of a second insert of the second coupling device, which is of same structure as the first insert and thus also having a fastening region, wherein in a pre-coupled state of the aseptic coupling arrangement, with two membranes interposed between the connector of the first coupling device and the connector of the second coupling device, the flange of the first coupling device and the flange of the second coupling device are interlocked in a flange-to-flange connection area where the first insert and the second insert extend,
wherein the interlocked flanges define at the external side walls thereof two opposite parallel surfaces for a first grasping of the aseptic coupling arrangement, preferably by one hand of an operator, while the sliders are also interlocked to provide a common pulling tab or gripper, graspable by the operator through a second grasping, preferably using the other operator's hand, to simultaneously perform the sliding disengagement of all the slider fingers.

Typically, each slide is provided between the two opposite parallel surfaces serving for the first grasping of the aseptic coupling arrangement. The two opposite parallel surfaces can each combine two surface portions distributed in the two flanges, with these two surface portions being possibly a pair of flush surfaces of the respective flanges. Optionally, no axially protruding part (not fixation means) is required/provided on these two opposite parallel surfaces.

The aseptic coupling arrangement is easy to obtain with the pre-coupled state. The graspable tab/gripper is available to ensure efficient guiding and removal of the two membranes, without requiring the operator to have any experience regarding the pulling gesture. Flexibility exists for the way the external section is constructed, for instance: either with a hinge arrangement providing a movable/rotatable cover covering initially the membrane and the connector front end, or formed as a single piece/part extending from a membrane attachment area to a free end of a handle assembly. The hinge can possibly be fitted to the slide or stabilized in suitable manner in a spread configuration of the cover that becomes a part of the gripper.
More generally, the operator can quickly obtain the aseptic coupling arrangement, which comprises a first connecting device and a second connecting devices (identical connectors), typically interlocked using the male and female connecting members distributed in the two connectors (which can be genderless connectors). Thanks to the removable closure devices consisting of the membranes and the sliders, a connection system can be formed for fluid connection, after removal of the two membranes, without membrane deviation along the flange front surface and possibly without any rotation involved for the connectors after engaging the coupling parts of these connecting devices.

Any clipping interface, forming the female member to allow locking a pre-coupled state of these connectors with each membrane still present, can be integrated in such coupling arrangement. The flange of the connector can house each finger of the slider so that the only part of the slider that is graspable/accessible is the gripper/graspable portion that is arranged on only one side with respect to the external side wall. The female connecting member is adapted to contact the mating insert of the male member, typically engaged through an axial access. The insert may allow unlocking a final position of the slider, so that in a pre-coupled state (ready state for the membrane pulling action) the slider is in a modified inserted position.

In some preferred options, the slider of the first connecting device includes a resilient member having or forming a latching part and is a first slider configured to latchingly engage an abutment surface (in the flange) during the sliding engagement of the first slider, before obtaining the pre-coupled state, to obtain a first snap fit configuration that prevents the sliding disengagement of the first slider,
wherein the slider of the second connecting device includes a resilient member having or forming a latching part and is a second slider configured to latchingly engage an abutment surface (in the corresponding flange) during the sliding engagement of the second slider, before obtaining the pre-coupled state, to obtain a second snap fit configuration that prevents the sliding disengagement of the second slider,
and wherein the latching parts are configured to be pushed, preferably axially, in opposite directions and unlocked by the first insert and the second insert, respectively, when obtaining the pre-coupled state so that the first snap fit configuration and the second snap fit configuration do not apply anymore, whereby the sliding disengagement of all the fingers (slider fingers) is allowed in the pre-coupled state.

With such arrangement when connecting the two aseptic connectors, this prevents risk/possibility to have an incomplete connection/interlocking of the assembly (for instance, either the snapping is incomplete or only one fixture is established instead of two fixtures). Indeed, the operator cannot remove the membrane while the connection is at risk or reopening. Practically, the assembly of the two complementary connectors cannot bend and there is no risk of partially disconnecting any sealing contact and breach asepticity.

Each of the first aseptic coupling device and the second aseptic coupling device is a connecting device such as above presented. In the aseptic coupling arrangement, in a pre-coupled state of the aseptic coupling arrangement (with two membranes interposed between the connector of the first aseptic coupling device and the connector of the second aseptic coupling device), the flange of the first aseptic coupling device and the flange of the second aseptic coupling device are interlocked in a flange-to-flange connection area, which is an area where the first insert and the second insert extend, the connection area being an annular connection area crossed by:
- one or two fingers of a first slider, which is the slider provided in the first aseptic coupling device; and
- one or two fingers of a second slider, which is the slider provided in the second aseptic coupling device.

With such configuration, a compact connection system is obtained, while advantageously having two sliders facilitating management of the connection (with orthogonal arrangement of the slider fingers, as compared to the inserts, and as compared to the pulling direction) and preventing any unsuitable closing portion deviation. For the locking of the aseptic coupling arrangement, kept after the membrane removal, the connectors directly interlock in the pre-coupled state, preferably without any additional action involved by the user to actuate the sliders, so that no additional gesture is required. When the connectors are genderless, the slider assembling step can be facilitated before the step of obtaining the pre-coupled state. Each slide may have a similar or identical way to be engaged. Besides, using identical sliders, the unlocking of the slider inserted positions by a pair of the mating connecting members can be obtained simultaneously, when achieving the axial insertion of the first and second inserts provided in the male connecting members.

In preferred embodiments, the sliders are not involved for the locking action between two connectors. In the coupling arrangement, the first slider and the second slider, distributed in the connecting devices, typically comprise each a U-shape with same orientation (the free ends of the fingers involved in the U-shaped are arranged away from the common graspable portion). They can also extend parallel, sharing same orientation, while the two external sections are fixed/combined at a same actuation side, these external sections being distributed in the first slider and the second slider, for allowing (for an unclipped state of the inserted/inner parts of the sliders) a single pulling action for pulling the two membranes.

The flange can have a cavity of hollow guiding part with a side opening to provide a side access to the locking region involved for retaining the corresponding slider, namely the region where a snap fit configuration or similar engagement using a detent member/surface is obtained. A finger of the slider, possibly extending straight along a transverse direction, is configured to have two inserted configurations inside the cavity: one for a locking or snap fit configuration, and another one compatible with sliding disengagement. The hollow guiding part may be a peripheral cavity, not interfering with the interior space.
In preferred embodiments, the slider has two parallel fingers that are elongated (possibly each longer than the external diameter or size of the annular sear), cooperating with two hollow guiding parts of the flange.
In the pre-couplded state, the slider can keep inserted position but with an unlatch configuration so that the slider is removable upon pulling the external section. In the unlatch configuration, the latching part of the slider, which a flexible member, can be depressed radially inward.

In some examples, a branch or member of the slider (a flexible tab or flexible section of a finger), resiliently deformable to form the latching part, possibly extending straight along a transverse direction, is configured to be pushed inside the cavity without any modification of the position/any translation of the slider along the pulling direction. The configuration obtained via this pushing can be a configuration where the slider member/tab extends between a pushing member/surface of the insert and an end of the cylindrical wall delimiting the interior space. In such option, the (flexible) latching part of the slider may be depressed axially rearward (away from a junction plane of the connection system, being pushed rearwardly). Due to the fingers that are fitted in the flange, each slider remains fully inserted in the slide in absence of any pulling action exerted at the external sections. Use of a bulged part in a part of the slider that is arranged between the two fingers can also help for stabilizing the slider that is kept in the inserted and unlocked position.

### BRIEF DESCRIPTION OF THE DRAWINGS

The figures of the drawings are now briefly described.

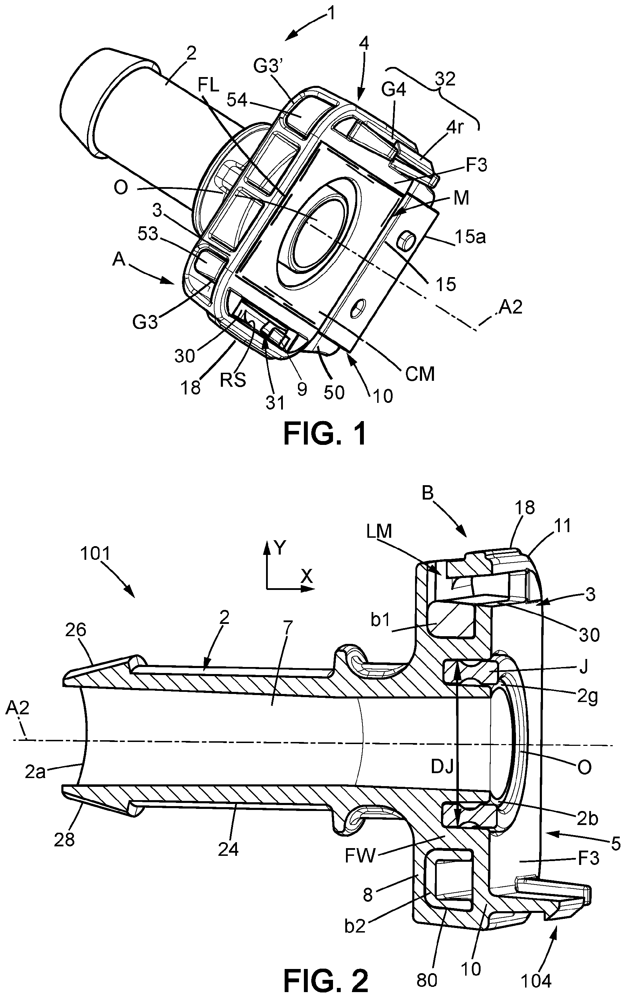

Fig. 1 is a perspective view of an exemplary fluid connecting device before use, with associated membrane for covering a connector opening illustrated away from its sealing position.
Fig. 2 is a longitudinal cross-sectional view showing, after removal of a membrane, structure of a connector of same type as in the embodiment of Fig. 1, this connector being genderless.

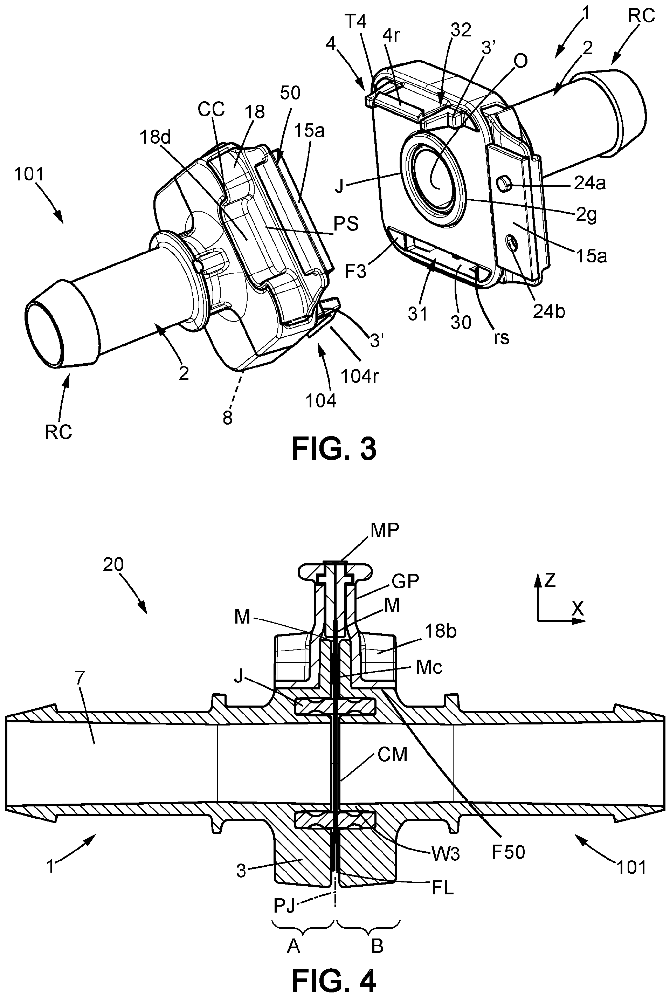

Fig. 3 is a perspective view of the two parts of an aseptic coupling arrangement, including the fluid connecting device of Fig. 1 and a compatible identical connecting device, each having same attachment structure with a corresponding membrane removable by a pulling action involving a slider with insertion finger(s).
Fig. 4 is a longitudinal cross-sectional view of the coupling arrangement of Fig. 3, along a longitudinal plane that includes a central axis of this arrangement, in a pre-coupled state in which a connection system is obtained with two identical connectors mutually fastened, before removal of the aseptic membranes.

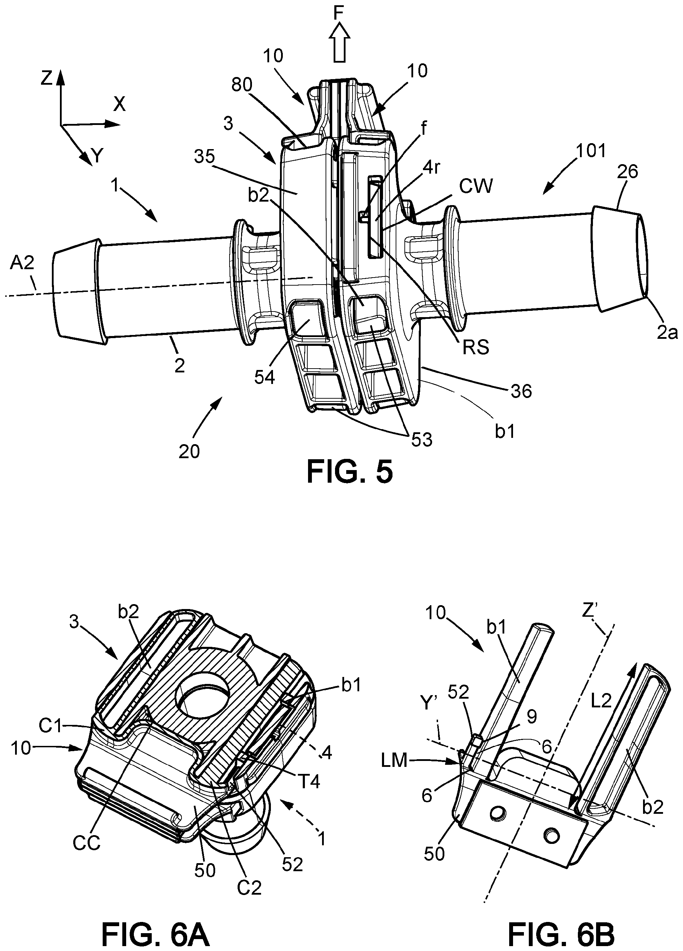

Fig. 5 illustrates a coupling arrangement, possibly the same as the one of Fig. 4 with a pair of sliders assembled to allow a single pulling action in the pre-coupled state obtained by locking means housed in the flanges of the connectors.
Fig. 6A is a perspective view with a cut, illustrating integration of a slider or sliding piece in the connecting device, using a slide with two spaced guiding parts according to an embodiment, the sliding piece also including a latching part involved for reaching a snap fit configuration of the slider.
Fig. 6B illustrates an exemplary sliding piece forming the slider, according to a first embodiment.

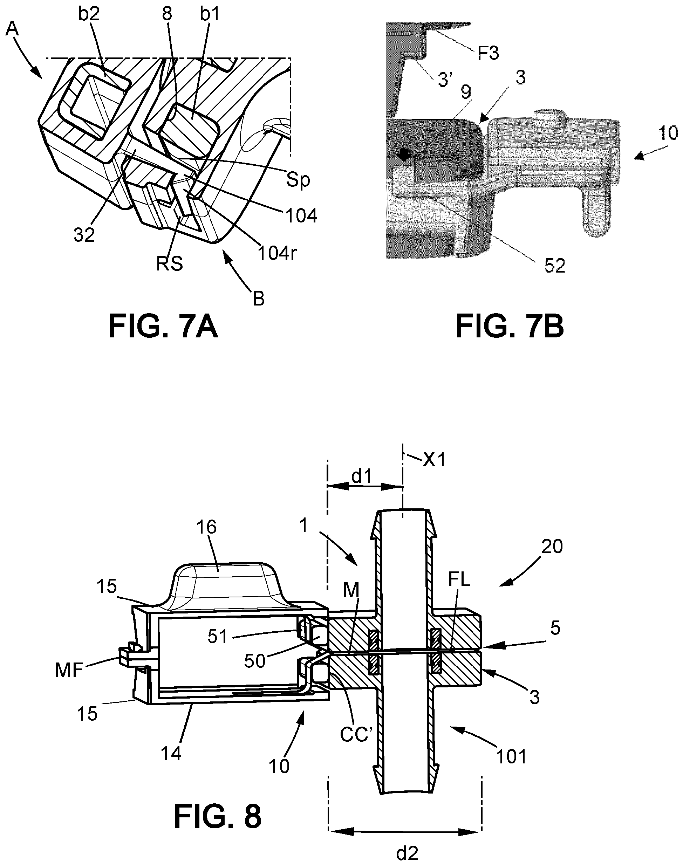

Fig. 7A shows a detail (using a cut view, parallel to a longitudinal axis) of a flange-to-flange connection, illustrating fully inserted position of a slider finger received in the slide of a flange to be adjacent to a male insert of the other flange, once this insert is engaged in the complementary female connecting member.
Fig. 7B is a detail view obtained just before the pre-coupled state of Fig. 7A, with an axial surface portion of the male insert in alignment with the axial access for pushing a latching part provided on the slider, along a pushing direction parallel to a longitudinal axis, so that a proper snapping of the male insert can cause disengagement of a snap fit that was obtained for the slider (slider such as shown in Fig 6B).
Fig. 8 illustrates, along a longitudinal plane, a pre-coupled state of an aseptic coupling arrangement with two interlocked connecting devices, here with a second embodiment of the sliders, which carry each a hinged cover at an external side at the opposite from the one or two fingers received in the flange slide.

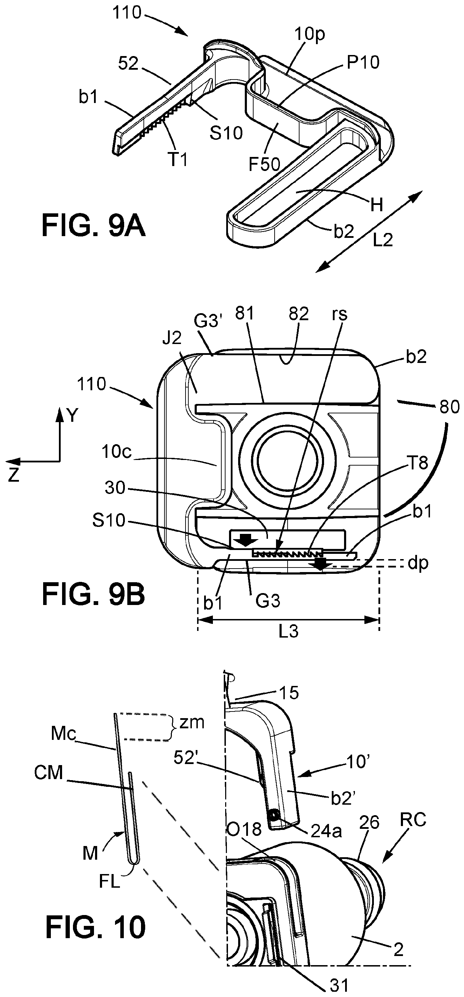

Fig. 9A is a perspective view of a one-piece slider according to a third embodiment (possibly compatible or adaptable with use of a gripper or gripping portion like in the first embodiment or second embodiment), using a toothed section that provides an anti-return effect.
Fig. 9B illustrates by a cross-section view a connecting device including the slider of Fig. 9A engaged in the slide provided in the flange, here with an empty area adjacent to the finger having the toothed section to allow a radial pushing of this finger.
Fig. 10 illustrates some parts of a connecting device according a variant, also with the flange suitable to guide the membrane pulling by guiding a slider, here with the slider that includes a finger configured to slide along an external face of the flange, for instance with a latching part formed on this finger to reach a snap fit configuration of the slider.

### MORE DETAILED DESCRIPTION

A detailed description of several embodiments of the invention is provided below, accompanied with examples and with reference to the drawings.

In the various figures, the same references are used to designate identical or similar elements.

Referring to Figs 1-2, it is shown an exemplary construction of a connecting device that can be considered as sterilized, thus being treated by suitable sterilization technique, including gamma irradiation, E-beam, ethylene oxide (EtO) and/or autoclave technologies. The connecting device comprises a fluid connector 1, 101 and a membrane M forming all or part of an aseptic attachment structure 5. The membrane M, which may include a fold, closes an opening O to the interior passage 7 of the connector 1, 101. The membrane M has a closing portion CM bonded to a connector front face surface by a heat bond or by any suitable continuous annular fixation ensuring asepticity, the membrane M being removably bonded (using peelable membrane material). The membrane M is pullable by providing a pulling portion or handle part. The fold may be a folding line FL opposite to the pulling portion.

The connector 1, 101 made of a rigid plastic material for instance, comprises a main body, which is a body 2 of tubular shape, suitable to delimit an interior space 7. The tubular body 2 can delimit a fluid passage of constant section, possibly with an inner cylindrical surface for delimiting the interior space 7. The tubular body 2 is provided with a flange 3 and may extend between a first open end 2a and a second open end 2b that is forming an opening O at a flange front face F3. The connector 1 may have a longitudinal axis A2, around which the tubular body 2 longitudinally extends. A cylindrical inner face may delimit the interior space 7, with this longitudinal axis A2 possibly being a central/symmetry axis of the tubular body 2.

As apparent in Figs 1-2, the opening O may be a central opening arranged between a female connecting member 31 and a male connecting member 32 that are carried by the tubular body 2. The female connecting member 31 may be a flush mount member of the flange 3 (forming a recess in the flange 3). The members 31, 32 may, at least partly, be each included in a same single piece that forms the tubular body 2, with the flange 3 thus included as a peripheral part of the tubular body 2. The flange front face F3, forming for instance a planar face, has an axial access 30 of the female connecting member 31, while the male connecting member 32 protrudes axially from the flange front face F3. An insertion channel can be formed by the female connecting member 31, at the rear of the axial access 30.
Figs 1-2 show complementary connectors 1, 101 that are genderless, so that they may be identical. In other embodiments, one of the connectors may carry/include the male member(s), while the other connector is provided with the complementary female member(s). These interconnecting members can be provided peripherally/around the sealed area where the membrane M adheres to the flange front face F3.

Referring to Fig. 1, the membrane M can be removably attached to the connector 1 to (partly) cover the flange front face F3 and seal the opening O. The membrane M may have a closing portion CM coupled to the front face of the tubular body 2, extending completely around the opening O and beyond this opening O at an annular attachment area. The closing portion CM is also covering a seal J that extends annularly around the opening O. An annular bond is provided for removably fixing the membrane M, with the annular bond encircling the seal J or similar annular seal element
This seal J can be received in a groove 2g that opens forward. A pull portion can be provided so that the membrane M is removable by a pulling action exerted away from the second open end 2b. The removable membrane M prevents bio-contaminants and/or fine particles from passing through. The membrane M may be porous for gas.
The membrane M may be supplemented with a stiff member, possibly perforated or provided with a recess for a coupling with a compatible (identical) stiff member provided for the other membrane M of the compatible connecting device.

As schematically illustrated in Figs 4 and 10, the membrane M can have two areas fixed at different parts of a connecting device. On the one hand, the membrane M has a closing portion CM provided with an attachment surface that has a given length along Z direction (pulling direction), sufficient to cover the seal J and typically sufficient to adhere to the flange front surface around the seal J. The shape or outer perimeter of the attachment surface may be substantially square or rectangular. On the other hand, the pulling portion can have an offset fixation area zm (Fig. 10) offset relative to the closing portion (and possibly formed as an extension of a folded overlapping portion Mc). The offset fixation area may be pinched, for instance by a hinged part of a slider extension (forming a subpart of the handle part 15a). The offset fixation area allows the membrane M to be secured to a rigid gripper or similar handle part 15. As detailed below, this part 15 can included in or be under the form of an external section 50 of a slider 10, 10', 110.
A fold or folding line FL may be formed at a side, possibly forming a side of the attachment surface. In such case, the overlapping portion Mc (Fig. 4) can be arranged to cover the closing portion CM, to protect this closing portion CM while extending -along Z direction- from the folding line FL to the pulling portion.

Whatever the way the membrane M is adhered to the flange front face F3 and to the slider 10, 10', 110, the connector 1 is thus provided with an aseptic attachment structure 5 that provides a graspable portion for removal of the membrane M. Passage of the fluid conveyed through the fluidic connection using two interlocked connectors 1, 101 is allowed by having the interior spaces 7 of these connectors 1, 101 tightly communication at an interface formed with two identical seals, possibly identical seals J. The connector 1 also has a flange 3 that may extend peripherally around (all around, for instance) the cylindrical part of the tubular body 2.

### Exemplary structures of the flange in a connector

Figs 3-5 show an exemplary way of interconnecting two coupling parts A, B, here with connectors 1, 101 of the genderless type, without any rotation involved for instance. More generally the flange 3 may be suitable for an efficient interlocking between two compatible connectors 1, 101, having this flange 3 and typically carrying a same kind of membrane M. As apparent in Fig. 1, the membrane M can be of low thickness, so that in pre-coupled state (before removal of membrane) it can be sandwiched/pressed directly between the two seals J distributed in the connectors 1, 101 (see Fig. 4).
As shown in Figs 1-3 in particular -for a genderless conception- the flange 3 can be provided with a female connecting member 31 (offset on a given side) and a male connecting member 32 that is arranged on a different side of the flange, as apparent in the embodiment of Fig. 1 for instance.

An axial access 30 is provided at the female connecting member 31, possibly forming a through passage (axial passageway) from the face F3 to a recess where a clipping or similar engagement with axial abutment can be obtained. The axial access 30 may have a width (along Z-direction) and a height or radial size (along Y-direction as shown in Fig. 2), with the width superior to the radial size. This clipping or engagement is an action, at the end of the male connecting members insertion, to lock a connection between the two mating flanges 3, forming the coupling parts A, B of the connection system 100. The axial access 30 may open through the front face F3, to be adjacent to the covering portion CM.
Referring to Fig. 2, the insertion passageway that extends from the axial access 30 can progressively taper toward a rear wall of the flange 3 and/or have a progressive reduction in the radial size along Y-direction. For instance, the external side wall 18 has an inner face that is tilted to delimit this axial passageway, to thus reduce (progressively) a passageway size as measured perpendicular to Z-direction (pulling direction). Such tapering may provide a centering effect when inserting the male connecting member 32 through the axial access 30.

The flange 3 can have an external side wall 18 and optionally also an inner flange side wall W3 and is provided with a housing 8 for receiving and typically retaining the slider 10, 10', 110. The housing 8 opens on a given side of the flange 3, which is the side configured to allow the pulling of the membrane M, knowing that the membrane M is secured to the slider that can only move along a direction (Z direction) perpendicular to the longitudinal axis A2 and parallel to the closing portion M. The flange 3 has a hollow structure around the interior space 7, possibly with grooves or inner channels, adapted to form a slide 80 along Z direction, i.e. perpendicular to the longitudinal axis A2. It is understood that the slide 80 is provided as an integral part of the flange 3 and has a sufficient length to allow a control of the pulling of the membrane M by preventing any deviation of the slider 10, 10', 110 with respect to Z direction.

A guiding length of the slide 80 is greater than a diameter of the seal, which can be the external diameter DJ of the annular seal element/seal J, and sufficient to prevent, at least before the closing portion CM is away from the opening O:
- any rotation of the slider 10; 10'; 110 relative to the flange 3; and
- any slider deviation.

In other words, a guiding is maintained at slider-flange interface so that this guiding is maintained at the time of the gesture to pull the slider 10, 10', 110 that drives the removal of the membrane M, when performing a sliding disengagement (of the slider) and pulling of the membrane M, by a pulling action using the slider 10, 10', 110.

As shown in Figs 1, 6A-6B and 9A-9B, guiding length can be defined by insertion length of one or two fingers b1, b2, preferably at least by insertion length L2 of an elongated finger b2. Typically, a depth d2 of the slide 80 may substantially correspond to this insertion length L2. The insertion length L2 may represent between 70 and 80% of the flange size L3 as measured along the Z-direction/pulling direction.
The insertion length L2 or any suitable guiding length provided by the slide 80 may be at least equal to a size of the attachment surface of the closing portion CM, and possibly equal or similar to an entire width of the flange 3 or connector 1, 101. As a result, the slider 10, 10', 110 can deviate from the sliding direction only after the closing portion CM of the membrane has been displaced out of the annular sealing area created between the two seals J. The size of attachment surface can be slightly inferior to an external size of the flange, along the pulling direction.

The insertion length L2 can be defined/measured between:
- a finger junction with the external section 50, that is positioned at a flange side (of the external side wall 18) in contact with the external section 50,
- and a finger free end 54 that can reach a cavity end provided in the slide 80.

A configuration of the slider 10, 10', 110 with two fingers b1, b2, spaced and spread to have the interior space 7 passing in-between can be preferred, with these two fingers stabilizing the slider that can reach a retained position when the two fingers b1, b2 are properly/fully inserted in the slide 80. The insertion length L2 may also be superior to d1, where d1 is radial distance between the handle part 15 (part arranged beyond the external side wall 18) and the central axis X1 of the coupling arrangement 20 (this axis X1 defining a direction of the axial passage for fluid).
At least one finger, possibly the two fingers b1 and b2 may represent at least 60 or 75% of the flange size L3.

As illustrated, the flange 3 can have one or two guiding parts G3, G3' to maintain a guiding direction both for sliding engagement and sliding disengagement of the slider 10, 10', 110 that is removably engaged. Typically, a slider insertable part is received in cavities or guiding parts G3, G3' of the flange 3, which constitute all or part of the slide 80. The disposition with two parallel guiding parts G3, G3' in the flange 3 can be provided with one or two axial open ends of the slide 80, at the opposite from the graspable handle part 15 formed in the external section 50 of the slider 10. One or two fingers b1, b2 can have their free ends 53, 54 coming flush with the externa side wall 18 at the opposite from the external section 50, as in the option shown in Fig. 1.
The flange 3 can have a hollow structure suitable to receive fingers b1, b2, b2' of a fork structure provided by the slider 10, 10', 110. The external section 50 can have an extension or width along Y-direction (transverse direction perpendicular to the pulling direction /Z-direction) to allow engagement of two fingers distributed along a width of the flange 3.

The outer side of the side wall 18 may include a non-planar surface, preferably provided with a recess 18d (Fig. 3), at the side covered by the external section 50 to provide an additional guiding effect at the end of the sliding engagement. More generally, the external section 50 can laterally cover the flange 3 and is configured to rest against a seating surface provided at an external side wall 18 of the flange 3. The slider 10, 10', 110 can abut against this outer side of the flange 3 when reaching a slider retained position, such as illustrated for example in Figs 1 and 3.
The external section 50 can be provided with 3D-structure, for instance with a bordering wall P10 that contacts the flange 3 and extends transverse to the pulling direction. This bordering wall P10 is extended by the handle part 15 that can be flat and protrude/extend along a plane parallel to the pulling direction. The handle part 15 may define a recess or cavity between the bordering wall P10 and a relief or abutment bar 10p provided radially at a distance from the bordering wall P10. The radial portion PS of the handle part 15 allows a pinching and the external section 50 thus can have two outer surfaces that are adjacent and substantially perpendicular. The bordering wall P10 may form a junction with the first finger b1 and a junction with the second finger b2, at the opposite from the handle part 15.

The external section 50 has an inner face F50, provided between the junction with the two parallel fingers b1, b2, that can contact the external side wall 18. Optionally, this inner face F50 can be a bulged face, with a bulged part 10c protruding toward the interior space 7 and filling (entirely or partly) the recess 18d. The elongated finger b2 can be connected to the external section 50 at a junction J2 that is:
- at a distance from the bulged part 10c; and
- more distant from the opening O than the bulged part.

The flange 3, which can be molded from plastic material, can include an intermediate annular wall FW (Fig. 2) interposed - with a concentrical arrangement- between an inner flange side wall W3 (Fig. 4) and an external side wall 18. Such configuration may provide a compact flange 3 with:
- a groove 2g of annular shape for the seal J (at the front side), this front groove 2g being arranged between the intermediate annular wall FW and the inner flange side wall W3 that delimits the central opening O;
- and one or more hollow guiding parts G3, G3' of the slide 80.

One of the two guiding parts G3 can be radially spaced from the external side wall 18 so that a locking/receiving area of the female connecting member 31 can extend between the external side wall 18 and a finger b1 received in this guiding part. A relatively small spacing Sp (Fig. 7A) can be provided between the male connecting member 32 as inserted the finger b1, to allow a flexing of a resiliently deformable part of the male connecting member 32 involved for engagement with the retaining surface RS.
More generally, the flange 3 can have a construction with a housing 8 that can include, on a same side with respect to the interior space 7:
- a cavity of the female member 31 with a retaining surface RS for the coupling with a mating male connecting member 104,
- and, close this cavity, the guiding part G3 that typically also includes a detent member or abutment surface rs that can cause a latching part 52 of the slider 10, 10' to stop (axial retaining effect).

Since two guide rails or guiding parts G3, G3' are involved, possibly inside the flange 3 (typically inside a connector body formed as one piece), the slider 10, 10', 110 can be stably inserted in the flange 3 with a rectilinear sliding (translation movement). Such sliding engagement can be performed before or after fixing the membrane M to the flange front face F3. In some variants, the slider 10 can have only one insertable elongated finger, possibly with a radial outer protrusion (radial protrusion for a retaining effect, possibly shorter than the elongated finger) that can be formed on or secured to the flange 3 to cooperate with another sliding portion of the slider 10, which is a portion distinct from the elongated finger and optionally of annular shape to cooperate and follow a guiding direction of the radial protrusion.

### Exemplary integration of an anti-actuation effect preventing accidental pulling

The membrane M can have an end, proximal to the actuation area for the pulling, fitted with the pulling gripper/graspable part 15 that is a part of the slider 10, 10', 110 (integral part when the slider is a single piece of plastic material). This association causes the membrane M to follow movement of the graspable part 15, once the membrane M is secured to the slider 10, 10', 110. The flange 3 can include an anchoring region, with an abutment surface rs in non-limiting embodiments of Figs 1, 3 and 9A-9B; so that unwanted movement of the slider 10, 10', 110 is prevented: a retaining effect is ensured. Since the membrane M extends planar along the flange 3 and closes the opening O by the closing portion CM, immobilization of the slider 10, 10', 110' stabilizes the membrane M.

In other words, in any of the options to have a suitable guiding with elongated guiding length preventing slider deviation, the slider 10, 10', 110 can have an inserted position, in which it is retained without any possibility to pull the slider 10, 10', 110. This anti-actuation effect can be provided for the connecting device having its aseptic attachment structure, before any connection to a complementary connecting device.

The flanges 3 and the sliders 10, 10', 110 can be designed to have a latching part 52, 52' that engages with a suitable detent member or abutment surface rs, in order to have an anti-actuation effect.
An exemplary latching part 52, with a lug 9, can be seen in Fig. 6B as parallel short insert arranged parallel to the finger(s) of the slider 10. Fig. 1 shows position of lug 9 once the slider 10 has been engaged, reaching its inserted and locked position. In such situation the latching part 52 ensures a temporary locking, until the connector 1 is interlocked in a connection system with adjoined position of the membranes M. Indeed the latching part 52 is protruding inside the female connecting member 31 to be pushable during the interlocking of the coupling parts A and B.

More generally, in the connector 1, 101, each flange 3 can be constructed with an indirectly actuatable part, available to be displaced and allow the pulling of the slider 10, 10', 110. Such indirect actuation can involve an insert or a pushing surface in the male connecting member 32 of the complementary connector 101, 1, which such insert or tab 104 interacting with the slider (with the latching part 52, 52' provided in this slider) engaged in the flange 3. Before the coupling with the male connecting members 32 properly inserted, the sliders 10, 10', 110 are locked with a snap fit or similar configuration using a detent/abutment surface rs (see Fig. 3 and Fig. 9B) included in the flange 3.

This prevents actuation to pull the membrane M for each connecting device. Two options are illustrated, respectively in Figs 7A-7B and in Figs 9A-9B, here for connectors 1, 101 that are genderless. Fig. 10 also shows another option for a retaining effect, with the connector not necessarily being genderless.

Figs 1, 6A-6B and 7A-7B show a slider 10 having a fork structure with a first finger b1 and a second finger b2 and also a latching par 52 that is offset/more distant from the axis Z' corresponding to effective pulling direction when the slider 10 has been slidably engaged in the flange 3. In this first embodiment, the latching part 52 is a parallel tab, connected to the external section 50 and having the retaining lug 9 (Fig. 1) to ensure a snap fit. The slider 10 has a slot 6 separating the latching part 52 from the adjacent finger b1 to permit the latching part or tab to be flexed. When engaging the slider 10, the lug 9 can engage in a groove or recess as shown in Fig. 1. This recess is delimited by the abutment surface rs (Fig. 3), which may be a surface shifted, along Y-direction, relative to the guiding part G3 for guiding the first finger b1.

When obtaining the connection arrangement 20, as shown in Fig. 6A, the position of the latching part 52 in the connector 101, along the external side wall 18 of the flange 3, slightly deviates relative to the pulling direction/ Z-direction due to an axial pushing performed by the other connector 1, typically by a pushing surface 3' (Fig. 3) provided in the male connecting member 32. This pushing surface 3' can possibly be arranged in the inserting tab 4 or in a protrusion T4 provided parallel to the inserting tab 4.

In some options, the slider 10, 110 can have a stabilizing wall following a surface profile of the flange external side wall 18. The bulged part 10c shown in Fig. 9B or similar relief or profile can be provided to enhance available space for pinching (by fingers) and/or friction of the engaged inner parts/fingers of the slider 10, 110 and/or to enhance a guiding effect with ease to finalize the proper insertion of each finger b1, b2 or b2'. A relative wide finger b2 can also be included in the slider 10, 110, possibly with a hollow structure of this wide finger b2 (with one hollow H or two cavities delimited between two sides of the finger b2 that define the finger width). The corresponding guiding part of the slide may have two long guiding surfaces 81, 82, parallel to a side of the external side wall 18, to provide an elongated sliding contact simultaneously for the two parallel sides of the finger b2 (one side proximal to the interior space 7 and the other being a distal side with respect to the interior space 7).

Referring to Figs 9A and 9B, a latching part 52 can be directly included in a finger, which is optionally a first finger b1 thinner than the wide finger b2. More generally, in a connector 1, the slider 110 can have a latching part 52 that can be pushed by an insert of the other complementary connector 101, while belonging to a sliding finger of the slider. In this second embodiment for obtaining the locking of the inserted position, a flexible member is provided at an end or main part of the finger b1 and the abutment surface rs can be shifted radially outward relative to a passageway for a male connecting member 32 of the other flange 3. As in the case illustrated in Fig. 9A, the finger b1 can be configured to provide:
- a part of the guiding effect (with coaction with the second finger b2 of the slider 110, also guiding the slider, especially for the pulling of the membrane M)
- a direct clipping or similar locking, by this finger b1, in the slide 80 (inserted from a flange side) that is an inner slide.

The clipping or fastening with the slider 110 involves a progressive anchoring action, compatible with a flexing/ local displacement dp of a section of the finger b1 along a radial direction, due to a spacing provided along this finger b1, between the finger b1 and the external side wall 18 (on a side perpendicular to the side open for receiving the fingers b1, b2 of the slider). Here, a finger locking is obtained by a toothed section T1 (typically comprising ratchet teeth having same radial orientation outward, for instance toward the external side wall surface of a suitable guiding part G3) cooperating with a mating section T8, possibly also toothed. This mating section forms reliefs that constitute the abutment surface rs, possibly distributed in several surface areas where the finger b1 is stopped/anchored to prevent any accidental pulling of the slider 110.

In an intermediate region, between the external section 50 and the toothed section T1 of the finger b1, a finger section with a pushable surface S10 is provided that is thicker than the toothed section to provide a partial delimitation of the cavity of the female connecting member 31 in the first connector 1, where an insert 104 of the male connecting member 32 is received. As indicated by the black arrow, a radial displacement can be driven by the insert 104, so that the slider 110 and its finger b1 becomes free to slide for disengaging from the flange 3.

In this second embodiment and in slight variants thereof, it is understood that the anti-actuation effect remains until a male member is fixed by axially passing through or crossing the flange 3 by an axial displacement/insertion and acting to push (by this displacement), for instance radially outward or inward, a slider finger that:
- is already engaged in the flange and serves for controlling removal of the membrane M, and
- has sufficient flexibility, to be flexed toward a direction transverse to the sliding/pulling direction and become no more engaged with the abutment surface rs.

Other embodiments are available for the slider. For instance, Fig. 10 shows a slider 10' having at least one outer finger b2', elongated, which can overlap the external side wall 18 of the flange 3, using an access 018 of an external flange groove. The external flange groove defines one of the two rails or guiding parts of the slide 80. A similar anti-actuation effect (temporary effect, to be obtained before having the pre-coupled state) can be obtained by using a lathing part 52' in the outer finger b2' and/or in a finger b1 that can slide inside the flange 3. The unlocking can involve any suitable inserting tab/insert engaged (axially) through the flange 3, typically via the axial access of the female connecting member 31.
In the non-limiting option of Fig. 10, one or two fingers of the slider 10' can be configured as an external member, with the external section 50 remaining adapted to form a combined graspable portion GP. Also in such embodiment, gesture is simple and the membrane pull is efficiently controlled as a sliding action for the slider 10' cannot be obtained with a deviation of the closing portion CM of the membrane M, the guiding length being suitably elongated to allow a deviation only after the closing portion CM is out from the seal-seal contact area.

While this example is illustrated with a flange 3 that is laterally slotted, other configuration may be provided for having the slider 10' interacting with guiding parts, for instance with a slider finger cooperating with a rear guide of the flange 3. The slider 10' can move in the slide to reach a retained position, with the latching part 52' reaching the suitable (releasable) anchoring region provided in the flange 3. Here, unlocking or similar disengagement of the latching part 52' can be performed automatically, like in the other embodiments, by an unclipping action since final position of the connecting members 31, 32 can displace/disengage (and thus release) the latching part 52'.

The fact that the slider 10, 110 or 10' cannot be pulled without proper flange-to-flange connection is advantageous since it provides a proof of good fit when the sliders become disengaged.

### Exemplary attachment of the membrane

The aseptic attachment structure 5 is formed by fastening the membrane M to an annular surface of the flange 3 at the flange front face F3, around the central opening O. Such attachment surface is covered by the closing portion CM (here a planar member made of the material of the membrane M) that belongs to the membrane M. The covering portion CM may have a margin portion that typically contacts and/or extends around the annular seal J surrounding the opening O. A complementary portion of the membrane M, possibly of rectangular shape, may extend radially outward from the covering portion CM to an extension acting as pull end part (at the pull portion/handle part 15) that is accessible for the operator, i.e. provided beyond an external rim or external side wall 18 of the flange 3.

As apparent in Figs 1, 2 and 3, the flange front face F3 (essentially planar in referred embodiments) can be partly covered by the membrane M, with the female connecting member 31 and the male connecting member 32 distributed on either side relative to the membrane M. The male connecting member 32 protrudes from the flange front face F3 and may follow a curved section of the attachment surface and/or be adjacent to the covering member CM. As illustrated in Figs 2, 4 and 8 for instance, the flange 3 may have a thickness (axial extension) that is superior to a length (axial size) of the seals J surrounding the opening O. The (female and male) connecting members 31, 32 may be disposed close to the central opening O. Such arrangement can facilitate:
- a first grasping with the pulling side still available, this grasping being performed, at parallel surfaces 35, 36 of the external side wall 18; and
- the pulling to be exerted from one side of the flange 3 that is not covered by the operator's hand used for the first grasping.

The flange 3 may be included in the tubular body 2 due to a one-piece construction (molded piece preferably), typically using plastic material such as any suitable rigid thermoplastic material. As shown in Figs 1-2, the tubular body 2 may extend around the longitudinal axis A2 that extends across the opening O, while the flange 3 may carry one or more piece(s) peripherally, around the interior space 7 and typically beyond an inner flange side wall W3 that possibly delimits the interior space 7.
In the connector 1 of Fig. 1 and in the other illustrated connectors as well, the flange 3 is provided with the connecting members 31, 32 accessible at a front side, allowing a front connecting with another connector 101. Typically, each of these members 31, 32 is included in a single piece forming the flange 3 (and possibly also delimiting the interior space 7, by forming all or part of the tubular body 2).

The female connecting member 31 and the male connecting member 32 may be distributed on two opposite peripheral areas of the flange 3 that are not covered by the membrane M. More generally, a genderless aseptic connecting device is obtained, which allows mating engagement of the connector 1 (as shown in Fig. 1) with an identical complementary connector 101 (such as shown in Fig. 2) to have the fluid connection, provided that each membrane M interposed between the interior spaces 7 (fluid pathways) has been removed. Possibly before such removal (with each membrane still present, typically), a clipping interface forming the female member 31 can allow locking a pre-coupled state of the connectors 1 and 101. Figs 4 and 5 illustrates an exemplary connection system 100 in this pre-coupled state, obtained after clipping two inserting tabs 4, 104 or similar inserts forming the male connecting members 32.

Referring to Fig. 8, it can be seen that two handle parts 15 can be clipped to form a handle assembly that is hollow, with option to insert a finger between the two handle parts 15 when having these parts not adjoined along the junction plane PJ. A clipping system with two complementary end tabs can form the fixation means that lock a fixed configuration of the handle parts 15. Each handle part 15 contains a hinged cover 14 for the corresponding connector 1, 101, which allow covering the membrane M (in a closed state if the cover 14) before starting operations to obtain a system for fluid connection using the two connectors 1, 101. A friction or a removable fixture can be used to removably retain the cover 14 in a closed configuration, before displacing, typically rotating this cover 14 around the hinge 51 (provided in the external section 50) when there is a need for establishing the connection. A pull tab 16 can be arranged on a protection plate or similar covering portion of the cover 14, to facilitate pulling of the hinged cover 14 to have a 180° rotation, so that the membrane M no more covered/protected. The one or more fingers b1, b2 stay engaged with the flange 3 when performing such cover displacement step.
When having a hinge 51, operational steps remain similar to obtain the fluid connection. Additional steps are: rotation to open the pair of covers 14; clipping or similar mating operation for coupling the covers when also performing the connexion of the coupling parts A, B.

Independently from the way the handle assembly is formed, using the two handle parts 15, a method of connecting two connecting devices may successively comprise:
- providing membranes of the two connectors 1, 101 (in an uncovered state) so that the membranes are facing each other;
- mating of the connectors to obtain a pre-coupled state;
- releasing the pair of latching parts or any suitable lockers (snap fit tabs for instance) when performing the mating step, using male connecting members to change inserted configuration of the sliders 10, 110, 10'; and
- pulling the fingers b1, b2, b2' that have been released, by pulling the handle assembly gathering the two external sections 50 to remove the membranes properly, each closing portion CM being pulled and detached/peeled off along the junction plane PJ without deviation thanks to the guiding length provided by the flange slides 80.

The attachment structure 5 and the integration of the sliders 10, 110, 10' can be compatible with a proof of good fit, possibly with the use of a separate secondary locker fitted on its own to the flange 3, in variants without the latching part 52, 52'. Besides, in some options, each finger b1, b2, b2' can include a latching element, possibly with more than one release system involved to allow the pulling of the slider.

### Interface for connection between two connectors in pre-coupled state

In illustrated embodiments, the two couplings parts A, B are identical and provided in genderless connectors 1, 101. The coupling is obtained without rotation along the X-direction, this X direction being here parallel to the longitudinal axis A2 of each connector 1, 101. Each flange 3 is carrying a slider and also a membrane M (affixed to the slider) extending along a direction, here Z-direction, which is perpendicular to the longitudinal axis A2 and also substantially perpendicular to Y-direction (Fig. 5) along which the fingers b1, b2 or b2' of the slider 10 are distributed for a removable mounting on the flange 3. In the illustrated embodiments, except for Fig 8 and 9B, Y-direction may be considered as a left-right direction, while Z-direction can form a substantially vertical direction.

As illustrated in Figs 4-5, a coupling arrangement 20 is obtained when reaching the pre-coupled state with the locking established (each retaining surface RS being engaged in the housing 8). The inserting tabs 4, 104 are thus forming a first insert of the connector 1 (Fig. 1) and a second insert of the connector 101 (Fig. 2), which are here identical connectors so that the interior spaces 7 are joined and delimited by a fully cylindrical inner face of the coupling arrangement 20.

The pair of inserts, forming inserting tabs 4, 104 or similar axial protruding parts in the coupling parts A, B, may each have an engagement rim, due to presence of a groove G4 or a radial protruding lug. Each of the inserts provided at the male connecting members 32 extend inside and through the axial access 30 provided in the corresponding female connecting member 31. More generally for a given connector 1, 101 to be used in such connection system 100, at least one female connection member 31 may be involved for an axial retaining effect, thus maintaining a contact between the seals J of annular shape, which may be an axial contact when having connectors 1, 101 of identical or similar structure.
When reaching full insertion of the inserts of the male connecting members 32, a locking can be obtained using latching means LM (Fig. 2) that can be arranged at the rear of or inside each flange 3. Then, after such mechanical connection, the membranes M can be removed to obtain a fluid communication.

The mechanical connection uses such female connecting member(s) 31 that is provided with a retaining surface RS, this connection involving a corresponding insert provided by the male connecting member 32 that is engaged and locked in properly inserted position. The connection may correspond to a removable or permanent fixation. When being a removable connection, a quick disconnection is available, typically requiring unlocking the fixation means.

In some embodiments, the size and shape of each profile, for the inserting tab 4 and the axial access 30 may be matching so that the connector 1 can cooperate with an identical connector 101 having same male and female connecting members 31, 32. Figs 4, 5 and 8 show all or part of a connection system/ arrangement 20 with the two connectors 1, 101 coupled at coupling parts A, B that are of identical structure. Each coupling part A or B may include a flange 3 as above disclosed (using a slider 10, 110 directly retaining the insert), with the aseptic attachment structure 5. In such options, the retaining surface RS may extend at a side of the slide part/guiding part G3 (Fig. 1) and close/adjacent to a slider finger.

Referring to Figs 3-5 and 6A, the connecting devices (with the connector 1 and with the connector 101, respectively) each have an offset radial portion that is a handle part 15 included in the slider 10 to pull the membrane M: this offset radial portion thus interacts with the aseptic attachment structure 5. This handle part 15 is radially spaced from the tubular body 2. In assembled state of the two connectors 1, 101 (with locked position), the handle parts 15, forming the respective pull portions, may form a common graspable portion GP (Fig. 4) to control/facilitate removal of the membranes M. The hoses/pipes or devices may be connected to the connection system/arrangement 20 obtained with such graspable portion GP extending radially in the pre-coupled state of the connection system, with the junction plane PJ intersecting this graspable portion GP.
Fastening means MF (Fig. 8, for instance formed as clips on hinged cover parts) and/or attachment radial surface 15a (for instance provided with interlocking pin 24a or any suitable reliefs protruding axially for cooperation with snap receiver 24b) can be provided at each handle part 15, to ensure the graspable portion can move as a single block at the pulling operation.

It is understood a fastening of two adjacent sliders 10, 10' can be obtained, when mating the connectors 1, 101. At the time of conceiving the aseptic attachment structure 5 of the connecting device 1, 101, the membrane M can be affixed to the flange front face F3 to seal the opening O and also be attached to the gripper, pull tab or cover 14 forming the actuatable handle part 15 that is pulled to displace the membrane M once the pre-coupled state is obtained. The membrane M can be firmly secured to this gripper.

The locking may involve a retaining action by abutment engagement between the insert (each insert typically) of the male connecting member 32 at a flexible member thereof (the tab that can be flexed) and the retaining surface RS provided in the female connecting member 31. This locking may be obtained for genderless connectors 1, 101 or for other connectors also having a transverse slide 80.

When using a radial protruding lug 4r or 104r, the proper axial abutment is easily obtained, with engagement against a retaining surface RS surface of the flange 3 when the insert tab extends beyond the axial access 30 in the female connecting member 31 to allow the membranes M to be in mutual contact. Optionally:
- position of the retaining surface RS may be intermediate between the external side wall 18 and the intermediate wall FW (Fig. 2),
- and/or a sliding displacement of at least one of the fingers b1, b2 of the slider 10, 10', 110 is performed closer to the longitudinal axis A2.
As apparent in Fig. 5 (here for the connector 101 in this example), a check window CWcan be provided at the external side wall 18 to allow checking that the protruding lug included in the connector 1 is properly engaged. Each connector 1, 101 may have such check window CW.

### Method of joining two connectors

Referring to Figs 1-2, Fig. 4 and Fig. 8, the method can involve fluid coupling components A and B that are identical, each provided in a connector 1, 101 of similar or identical structure, here genderless. The opening O may be a central opening, so that it may provide an axial passage, through the flange 3 and extended (rearwardly) by the tubular body 2. This axial passage is rotationally symmetrical about the longitudinal axis X1, allowing passage of the fluid conveyed through the fluidic connection.

This method firstly involves forming a coupling arrangement 20 (with an aseptic connection, possibly in a non-aseptic environment), comprising a first aseptic coupling device and a second aseptic coupling device, with the connectors 1, 101 interlocked at the coupling parts A, B. Typically, each connector 1, 101 has been preliminary fitted, at the open end 2a, with an associated tube (a tubing, a hose or the like), possibly using a barbed nozzle (with barb 26, possibly involving a clamp). In some options, a specific equipment is already provided with the tube. In some embodiments, a rear end of the connector 1, 101 may be also provided with a genderless connector. Besides, a step of sterilizing the connectors 1,101 and the aseptic attachment structure 5 can be performed, for instance with each of the connecting devices being:
-- provided with a corresponding slider 10, 10', 110 in fully inserted position,
- axially covered at least at the flange front face F3 by a corresponding membrane M of the attachment structure 5 with a membrane-slider connexion at an appropriate fixation area zm.

The following steps may be performed to achieve sterile joining of the two connectors 1, 101, although not necessarily in the order described:
- aligning the inserting tabs 4, 104 or similar inserts with facing axial accesses, by adjusting angular position of the flanges 3 of the connectors 1, 101;
- displace the sterilized connectors axially (X-direction), to have a flange-to-flange connection with each insert of the male connecting members 32 fully inserted through the corresponding axial access 30 of the female connecting member 31, so that the membranes M are adjacent with each other (with a contact between the overlapping portions Mc when having a fold for each membrane M);
- obtaining the pre-coupled state of the connection system, thus obtaining the coupling arrangement 20, such as illustrated in Figs 4-5 for instance, i.e. with the membranes M extending along a junction plane PJ;
- removing, simultaneously, the protective membranes M from the connectors 1, 101, by exerting a pulling force on a common pull portion (typically a graspable portion GP) to immediately obtain axial contact between the seals J (O-rings), without any relative rotation between the flanges 3.

Then fluid connection is achieved (with possibly a removal or releasing of some clamps provided adjacent to the connector(s), outside the coupling arrangement 20).

In the pre-coupled state, two membranes M are extending parallel and in mutual contact, before a combined removal of these membranes M. While hose nozzle with at least one barb 26 or similar connecting male member has been illustrated at the rear end of each connector 1, 101 or at a rear of a fitting RC, it is understood than any other connecting part may be used, possibly with the connector directly welded on a pouch, a bioreactor or any device used in a circuit where a biopharmaceutical fluid can flow.
From a control point of view for a "single use" system, each connector 1, 101 in the connecting device is compliant with starting a connection, which is aseptic. The genderless structure allows using the connecting devices (with connector 1, 101), with formation of the coupling arrangement 20, at several interfaces of a biopharmaceutical circuit.

Referring to Figs 1-2, 5 and 6B, the width of each inserting tab 4, 104 may be superior or equal to depth of the groove 2g. At least one bevelling may be provided at a free end of the inserting tab 4, 104, to help in having a guided insertion, simultaneously, of the inserting tabs 4, 104, in opposite directions. The locking is also performed simultaneously and automatically. At the locking area, the axial access 30 opens for the passage of a fastening region 4r, 104r orientated radially outward on each inserting tab end.

Then, after mating the coupling parts A, B, using the aseptic attachment structure 5, an aseptic connection can be made between the two connectors 1, 101. After removal of the membranes M, with the seals J in mutual contact, there is no requirement for the user to rotate or actuate any driving part to further displace or deform the flanges 3 and the seals S. In some variants, at least one of the connectors 1, 101 used in the fluid connection may be arranged with a plug, for instance with a plug that is spring biased or driven by a bayonet connection.

It is understood that, once the membrane(s) M is no more present due to the pulling action, the interlocking action by the connecting members 31, 32 is maintained and an axial pressure is exerted on each O-ring or seal J, thus ensuring a sealing function around the central passage defined at the openings O. The sealing elements or seals J may have an excess in length to ensure an axial pressure is exerted in such annular contact area, forming an annular junction.

The insertion movement for the interlocking can be completed when the fastening region 4r, 104, possibly designed with a tab (protruding radially outward for instance) cooperates with the retaining surface RS provided in the female connecting member 31, which means an abutment part or surface is engaged in the groove G4 that forms all or part of the fastening region 4r, 104r. Fig. 7A shows that the inserting tab 104 (idem for inserting tab 4), passing through the axial access 30, can thus be interposed radially between the intermediate wall FW and the outer side wall 18. As mentioned above, options to release the anti-actuation effect can be obtained by pushing an elastic-return part or latching part 52, 52' that serves to prevent radial displacement/any sliding disengagement before the coupling between the connectors. This pushing is typically performed by the male connecting member 32, either at the tab region or at an abutment/pushing surface 3' (provided at a shoulder part in the option of Figs 3 and 7B).
One or two triangular protrusions T4 may be provided in the male connecting member 32, these triangular protrusions T4 being rigid while the tab 104 or 4 can be radially flexible, with each tab 4, 104 possibly arranged between two triangular protrusions T4. Referring to Figs 3 and 7B, the pushing surface 3' in this male connecting member 32 can be provided in such protrusion T4 to allow pushing the latching part 52, 52' and unlock the slider 10 retained in the flange 3 forming the female connecting member 31.

Referring to Fig. 6B, the external section 50 can have a symmetry axis Z' that is a central axis of slider 10, such axis Z' defining a length direction of the slider 10. The two fingers b1, b2 extend along direction of this axis Z'.

Use of guiding parts to form a slides 80, distributed in the connectors 1, 101 of the connection system such as shown in Figs 5 and 8, allows forming relatively flat flanges 3 since each slide 80 extends in a transverse plane YZ perpendicular to the longitudinal axis X. The slider 10, 10', 110, possibly formed as a molded plastic part comprising a plate and latching part 52, 52' included in a finger b2' or parallel to such finger b2, may be a flat piece (compact piece along longitudinal axis X), not interfering with the interior space 7.

The tubular body 2 can be formed of plastic materials biocompatible with biopharmaceutical substances, for example polypropylene, polyethylene, polycarbonate, polysulfone, polyvinylidene chloride, polyethylenimine. The open end 2a can optionally be provided in a suitable fitting RC of a tubular body end 28 (Fig. 2), for instance molded with the flange and having at least one hose barb(s). It can also be provided in a clippable interface adapted to permanently clip the connector 1, 101 to a compatible component (assembled component). Alternatively, the open end 2a can be arranged directly in a flange structure compatible or identical to the structure of flange 3. It is understood the connector 1 with the coupling part A formed at the flange 3 can be adapted to be assembled to any component used for fluid transfer, possibly by including a coupling part at the rear end provided with the open end 2a.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

While the membrane M has been shown with two opposite edges parallel to the sliding direction, it is understood the design or geometry of the membrane M can be adapted. The membrane M can be provided with a folding line FL (Fig. 1), rectilinear or not, opposite from the pulling side. Due to this folding line FL and the substantially stretched or unfolded configuration of the overlapping membrane portion that covers the closing portion CM, pulling of the slider 10, 10', 110 that disengages from the slide 80 immediately causes the closing portion CM to be detached from a distal side (folding line side) that is distal with respect to the external section 50 of the slider 10, 10', 110.
- a sealing/closing portion adhering with an annular surface provided at the front of the connector, so that the connector opening is closed in aseptic manner by the closing portion,
- and an overlapping membrane portion, overlapping the closing portion and extended by a pulling part provided at the pulling side.

The flange 3 has been illustrated as having two parallel walls, parallel to the plane YZ, forming a front wall and a rear wall of the flange. However, in some variants, the flange 3 can have an additional compartment with an additional wall forming a partition. For instance, an additional locking piece or assembly can be provided to prevent accidental disconnection of the coupling involving the male and female connecting members 31, 32.
A locking ring or similar piece, can have transverse disposition, with a sliding mounting for instance to allow a clipping or snapping action enabled by each of the two additional pieces. A return part, included in this piece, may exert a return effect along a radial direction. When having an external push-button/actuator or similar external actuator, a pushing can be actuated from a side of the connector 1, to initiate/allow disconnection. When this piece is locking ring, the guiding and/or actuation of this piece may be as disclosed in document US 10487967.

## Claims

1. A connecting device for fluid connection, having a connector (1) and a membrane (M), the connecting device comprising:
- a tubular body (2) extending around a longitudinal axis (A2) of the connector and delimiting an interior space (7), the tubular body having an opening (O) at a front of the tubular body (2) where the interior space (7) axially opens;
- a flange (3) formed peripherally on the tubular body (2) around the interior space (7), the flange (3) comprising a female connecting member (31) and/or a male connecting member (32) protruding axially from a flange front face (F3) to provide a first insert having a fastening region (4r);
- an aseptic attachment structure (5) including the flange front face (F3) and the membrane (M) that is removably attached to the connector (1) at the flange front face (F3) to seal the opening (O);
- an annular seal element (J) providing an annular contact surface at the flange front face (F3), radially interposed between the opening (O) on the one hand, and the female connecting member (31) and/or a male connecting member (32) on the other hand; and
- a slider (10; 10'; 110) mounted on the flange (3) to be movable relative to the flange, along a direction (Y; Z) transverse to the longitudinal axis (A2).
wherein the membrane (M) is secured to an external section (50) of the slider (10; 10'; 110) and comprises a closing portion (CM) axially covering and sealing the opening (O);
wherein the slider (10; 10'; 110) is in a predefined engaged position relative to the flange (3) and comprises:
- the external section (50) that extends outside the flange (3) in the predefined engaged position; and
- one or two fingers (b1, b2; b2'), each rigid and protruding inwardly from the external section (50), parallel to the closing portion (CM) and perpendicular to the longitudinal axis (A2), allowing the slider (10; 10'; 110) to be retained, in said predefined engaged position, in a slide (80) that allows sliding engagement and sliding disengagement of said one or two fingers (b1, b2; b2') along a sliding direction perpendicular to the longitudinal axis (A2), the slide (80) being provided as an integral part of the flange (3),
and wherein a guiding length of the slide (80) is superior or equal to an internal diameter or external diameter (DJ) of the annular seal element (J) and sufficient to prevent any rotation of the slider (10; 10'; 110) relative to the flange (3) and any slider deviation at least before the closing portion (CM) is away from the opening (O), when performing the sliding disengagement and pulling the membrane (M) by a pulling action using the slider.

2. The connecting device according to claim 1, wherein the slide (80) comprises two guiding parts (G3, G3'), one of which being a cavity extending, inside the flange (3):
- from a mouth opening at a first side of the flange (3), allowing insertion of an elongated finger (b2; b2') that forms all or part of the one or two fingers,
- to a cavity end that extends at a second side of the flange, provided at the opposite from the first side, with the elongated finger (b2; b2') being longer than the external diameter (DJ) of an annular seal element (J) that surrounds the opening (O) to have a finger end (54) extending in the cavity end.

3. The connecting device according to claim 1, wherein the one or two fingers (b1, b2; b2') comprise an elongated finger (b2; b2') configured to be linearly moved in the slide (80) between two parallel guiding parts (G3, G3') of the slide (80), the elongated finger (b2; b2') being guided by at least one amongst a flange rear wall and a flange front wall that are parallel walls between which the two parallel guiding parts (G3, G3') extend to prevent any rotation and deviation of the elongated finger (b2; b2') during the sliding disengagement.

4. The connecting device according to claim 3, wherein the elongated finger (b2;
b2') is received inside the flange (3) in a cavity that extends from a mouth opening at a first side of the flange (3) to a cavity end that extends at a second side of the flange, the interior space (7) being interposed between the first side and the second side of the flange (3).

5. The connecting device according to claim 1, wherein the slider (10; 10'; 110) comprises two elongated fingers (b1, b2; b2') that extend parallel and are configured to be linearly moved in the slide (80), the interior space (7) extending between the two elongated fingers (b1, b2; b2').

6. The connecting device according to claim 2, wherein the slider (10; 10'; 110) comprises a first finger and a second finger, one of which is forming the elongated finger, so that the first finger and the second finger form part of a U-shaped stabilizing part of the slider, and wherein the slide (80) includes two rails for cooperating with the first and second fingers (b1, b2; b2').

7. The connecting device according to any one of the preceding claims, wherein the slider (10; 10'; 110) has latching means (52; 52') formed on or that co-act with the one or two fingers (b1, b2; b2') to lock and retain the slider (10; 10'; 110) in the predefined engaged position,
and wherein the latching means (52; 52') is deformable or displaceable, preferably without any sliding movement of the slider along the sliding direction, under a pushing action to unlock the slider (10; 10'; 110).

8. The connecting device according to any one of claims 1-7, wherein the connector (1) is genderless,
wherein the female connecting member (31) is provided with a locking region and an axial access (30), the axial access (30) preferably opening at the flange front face (F3),
and wherein the slide length is greater than a length of the male connecting member (32) as measured from the flange front face (F3).

9. The connecting device according to according to any one of claims 1-6, wherein the female connecting member (31) is provided with an axial access (30) and a retaining surface (RS) arranged inside the flange (3),
wherein the slider (10; 10'; 110) includes a resilient member having a latching part (52) configured to latchingly engage an abutment surface (rs) of the flange (3), which is a surface preferably provided within an inner cavity of the slide (80), to prevent the sliding disengagement,
wherein the axial access (30) allows insertion of a second insert, of same structure as the first insert and thus also having a fastening region (104r), the second insert being part of an identical complementary connector (101) genderless for the fluid connection and being sized and shaped for engagement of the retaining surface (RS) with the fastening region (104r) of the second insert, in a connection configuration,
and wherein in the connection configuration with the second insert engaged, the latching part (52) is pushed toward a disengaged insertion position allowing the sliding disengagement.

10. The connecting device according to any one of the preceding claims, wherein the external section (50) has an inner face (F50) that:
- extends between the two parallel fingers of the slider (10; 10'; 110);
- is bulged inwardly to form a bulged part (10c);
- laterally covers the flange (3) in the predefined engaged position;
wherein the inner face (F50) is in contact with the external side wall (18) at the seating surface that comprises a recess, while at least one contact area (CC) is obtained between the external section (50) and the seating surface, with the external section (50) of the slider (10; 10'; 110) having the bulged part (10c) filling or being engaged in the recess.

11. The connecting device according to any one of the preceding claims when depending on claim 9, wherein the retaining surface (RS) is configured to axially retain the second insert at the fastening region (104r) thereof in a locking configuration of the fluid connection, the second insert being formed in the complementary connector (101) that is suitable to be coupled to said connector (1) for enabling the fluid connection,
the locking configuration being obtained when the second insert passing through the axial access (30) is inserted in the female connecting member (31) to have a second insert end extending axially beyond the retaining surface (RS),
and wherein the latching part (52) is provided with a pushable surface (S10) located inside or adjacent to an insertion channel delimited by the female connecting member (31), whereby the disengaged insertion position is obtained upon pushing the pushable surface (S10) by the second insert to have the locked configuration of the fluid connection.

12. The connecting device according to any one of the preceding claims when depending on claim 9, wherein the slider (10; 10'; 110) is configured to be transversely moved, along a first direction perpendicular to the longitudinal axis (A2) with provision that the second insert has been inserted via the axial access (30),
and wherein the slider (10; 10'; 110) is snap fit by a finger lug (9) in an inner region of the flange (3), which is a region in alignment with the axial access (30) along a direction parallel the longitudinal axis (A2).

13. The connecting device according to any one of the preceding claims when depending on claim 8, wherein the connector (1) is a first aseptic coupling device for coupling to a second aseptic coupling device consisting in the identical complementary connector (101) and an associated membrane, the membrane (M) of the first aseptic coupling device being a first peel off membrane removably coupled to and at least partially covering the flange front face (F3) and the seal element (J), with the closing portion (CM) having a given attachment extension along the sliding direction
wherein the flange (3) of the connector (1), preferably formed in one piece with the tubular body (2), has along the sliding direction an external size superior to the given attachment extension and the guiding length of the slide (80) is superior or equal to the given attachment extension.

14. An aseptic coupling arrangement (20), comprising a first coupling device and a second coupling device, which are each provided with the connecting device according to any one of the preceding claims, the axial access (30) of the first coupling device allowing insertion of a second insert (104) of the second coupling device, which is of same structure as the first insert (4) and thus also having a fastening region (104r),
wherein in a pre-coupled state of the aseptic coupling arrangement, with two membranes (M) interposed between the connector (1) of the first coupling device and the connector (101) of the second coupling device, the flange (3) of the first coupling device and the flange (3) of the second coupling device are interlocked in a flange-to-flange connection area where the first insert (4) and the second insert (104) extend,
wherein the interlocked flanges (3) defines at the external side walls (18) thereof two opposite parallel surfaces (35, 36) for a first grasping of the aseptic coupling arrangement (20), preferably by one hand of an operator, while the sliders (10; 10'; 110) are also interlocked to provide a common pulling tab or gripper (GP), graspable by the operator through a second grasping, preferably using the other operator's hand, to simultaneously perform the sliding disengagement of all the slider fingers (b1, b2; b2'),
and wherein each slide (80) is provided between the two opposite parallel surfaces (35, 36) serving for the first grasping of the aseptic coupling arrangement.

15. The aseptic coupling arrangement (20) according to claim 14, wherein the slider (10; 10'; 110) of the first connecting device includes a resilient member having a latching part (52) and is a first slider configured to latchingly engage an abutment surface (rs) during the sliding engagement of the first slider, before obtaining the pre-coupled state, to obtain a first snap fit configuration that prevents the sliding disengagement of the first slider,
wherein the slider (10; 10'; 110) of the second connecting device includes a resilient member having a latching part (52) and is a second slider configured to latchingly engage an abutment surface (rs) during the sliding engagement of the second slider, before obtaining the pre-coupled state, to obtain a second snap fit configuration that prevents the sliding disengagement of the second slider,
and wherein the latching parts (52; 52') are pushed, preferably axially, in opposite directions and unlocked by the first insert and the second insert, respectively, when obtaining the pre-coupled state so that the first snap fit configuration and the second snap fit configuration do not apply anymore, whereby the sliding disengagement of all the fingers (b1, b2; b2') is allowed in the pre-coupled state.
